(19) **Europäisches Patentamt European Patent Office Office européen des brevets**

(11) **EP 3 954 276 B1**

(12) **EUROPEAN PATENT SPECIFICATION**

(45) Date of publication and mention of the grant of the patent:
**03.12.2025 Bulletin 2025/49**

(21) Application number: **20786748.2**

(22) Date of filing: **10.04.2020**

(51) International Patent Classification (IPC):
**A61B 5/00** (2006.01)     **A61B 5/1455** (2006.01)

(52) Cooperative Patent Classification (CPC):
**A61B 5/7275; A61B 5/0075; A61B 5/6814; A61B 5/7267;** A61B 5/14551; A61B 5/4064; A61B 5/4088; A61B 5/4878

(86) International application number:
**PCT/KR2020/004940**

(87) International publication number:
**WO 2020/209688 (15.10.2020 Gazette 2020/42)**

(54) **METHOD, SYSTEM, AND NON-TRANSITORY COMPUTER-READABLE RECORDING MEDIUM FOR ESTIMATING BIOMETRIC INFORMATION ABOUT A HEAD USING MACHINE LEARNING**

VERFAHREN, SYSTEM UND NICHTFLÜCHTIGES COMPUTERLESBARES AUFZEICHNUNGSMEDIUM ZUM SCHÄTZEN BIOMETRISCHER INFORMATIONEN ÜBER EINEN KOPF MITTELS MASCHINENLERNEN

PROCÉDÉ, SYSTÈME ET SUPPORT D'ENREGISTREMENT NON TRANSITOIRE LISIBLE PAR ORDINATEUR POUR ESTIMER DES INFORMATIONS BIOMÉTRIQUES RELATIVES À UNE TÊTE AU MOYEN D'UN APPRENTISSAGE MACHINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2019   KR 20190043379**

(43) Date of publication of application:
**16.02.2022   Bulletin 2022/07**

(73) Proprietor: **Korea Advanced Institute of Science and Technology Daejeon 34141 (KR)**

(72) Inventors:
• **BAE, Hyeon Min Daejeon 34141 (KR)**
• **JI, Min Su Daejeon 34141 (KR)**
• **YU, Seong Kwon Ulsan 44774 (KR)**
• **KOH, Bum Jun Daejeon 35200 (KR)**

(74) Representative: **RGTH Patentanwälte PartGmbB Neuer Wall 10 20354 Hamburg (DE)**

(56) References cited:
JP-A- 2009 101 057     JP-A- 2017 076 193
KR-A- 20180 001 299    KR-A- 20180 017 624
KR-A- 20180 099 434    US-A1- 2014 107 494
US-A1- 2017 231 501    US-A1- 2017 231 544
US-B1- 10 071 245

• FOURNIER MARC ET AL: "Realistic Head Model Design and 3D Brain Imaging of NIRS Signals Using Audio Stimuli on Preterm Neonates for Intra-Ventricular Hemorrhage Diagnosis", 1 October 2012, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 172 - 179, ISBN: 978-3-540-74549-5, XP047463858

## Description

TECHNICAL FIELD

The present disclosure relates to a method, a system, and a non-transitory computer-readable recording medium for estimating biometric information about a head using a machine learning.

BACKGROUND

Information about the biometrical state of a person's head portion obtained by monitoring the person's head portion (particularly, cerebral cortex) provides very important information in preventing, diagnosing, and treating stroke, cerebral edema, Alzheimer's disease, and the like, which are diseases associated with the person's brain.

Although methods such as MRI, CT, angiography, or the like are mainly used for the above-mentioned monitoring and the methods have an advantage of high measurement accuracy, such methods are expensive and provide only information about the measurement moment. Thus, there is a problem that continuous monitoring is difficult.

Near-infrared spectroscopy (NIRS) that has been introduced recently is a method of indirectly analyzing a bioactivity occurring in a body portion (e.g., brain or the like) of the person by measuring a degree of attenuation of near-infrared ray (due to scattering and absorption by oxidized or non-oxidized hemoglobin) which varies with a change in hemodynamic (e.g., concentrations of oxy hemoglobin and deoxy hemoglobin) which occurs in the body portion. The use of the NIRS to monitor the person' head portion has advantages in that it is inexpensive compared to the use of MRI, CT, angiography or the like and capable of continuous monitoring, but has a problem of low measurement accuracy. This problem is due to the fact that the conventional NIRS (Spatial Resolved Spectroscopy) analyzes an optical signal detected from the person's head on the assumption that an anatomical structure of the person's head is a homogenous structure, and as a result, the monitoring by the NIRS is necessarily affected by the biometrical state that are not related to the cerebral region such as the skin (e.g., a change in oxygen saturation of the skin).

In this regard, the present inventor(s) proposes a technique that makes an estimation model learn so as to estimate biometric information about a head of a person to be measured based on NIRS and in consideration of an anatomical structure of a head portion of the person to be measured, and analyzes an optical signal detected from the head portion of the person to be measured using the learned estimation model, thereby accurately and continuously monitoring the biometric information about the head of the person to be measured and increasing the cost-effectiveness of the monitoring.

[Prior Art Document]

[Patent Document]

Patent Document 1: Korean Laid Open Patent Publication No. 10-2016-0121348 (published on October 19, 2016)

US 10 071 245 B1 describes a system for mapping user behavior to brain regions of interest using a combination of cognitive-behavioral and functional-anatomical modeling.

The non patent literature "Realistic Head Model Design and 3D Brain Imaging of NIRS Signals Using Audio Stimuli on Preterm Neonates for Intra Ventricular Hemorrhage Diagnosis" of Fournier Marc et al describes an auditory stimulation and Near Infra-Red Spectroscopy (NIRS) hemodynamic changes acquisition protocol for preterm neonates.US 2017/231501 A1 describes the measuring of brain activity using super-pixel detection with wearable diffuse optical tomography.

SUMMARY OF THE INVENTION

The present invention is defined by the appended claims.

OUTLINE OF THE DISCLOSURE

An object of the present disclosure is to solve the aforementioned problems in the related art.

Another object of the present disclosure is to acquiring an analysis target optical signal detected from a head portion of a person to be measured by at least one optical sensor disposed on the head portion of the person to be measured, and estimating biometric information about the head of the person to be measured by analyzing the acquired analysis target optical signal using an estimation model learned based on data about an anatomical structure of at least one head portion, data about a biometrical state of the at least one head portion, and data about a optical signal associated with the data about the anatomical structure of at least one head portion and the data about the biometrical state of the at least one head portion.

Yet another object of the present disclosure is to provide a technique that makes an estimation model learn so as to estimate biometric information about a head of a person to be measured based on NIRS and in consideration of an anatomical structure of a head portion of the person to be measured, and analyzes an optical signal detected from the head portion of the person to be measured using the learned estimation model, thereby accurately and continuously monitoring the biometric information about the head of the person to be measured and increasing the cost-effectiveness of the monitoring.

Representative configurations of the present disclosure for achieving the above objects will be described below.

According to one aspect of the present disclosure, there is provided a method of estimating biometric information about a head using a machine learning, the method including: acquiring an analysis target optical signal detected from a head portion of a person to be measured by at least one optical sensor disposed on the head portion of the person to be measured; and estimating the biometric information about the head of the person to be measured by analyzing the acquired analysis target optical signal using an estimation model learned based on data about an anatomical structure of at least one head portion, data about a biometrical state of the at least one head portion, and data about a optical signal associated with the data about the anatomical structure of at least one head portion and the data about the biometrical state of the at least one head portion.

According to another aspect of the present disclosure, there is provided a system for estimating biometric information about a head by using a machine learning, including: an analysis target optical signal acquisition unit configured to acquire an analysis target optical signal detected from a head portion of a person to be measured by at least one optical sensor disposed on the head portion of the person to be measured; an estimation model management unit configured to make an estimation model learn based on data about an anatomical structure of at least one head portion, data about a biometrical state of the at least one head portion, and data about a optical signal associated with the data about the anatomical structure of at least one head portion and the data about the biometrical state of the at least one head portion; and a biometric information estimating unit configured to estimate the biometric information about the head of the person to be measured by analyzing the acquired analysis target optical signal using the learned estimation model learned.

In addition, there are further provided other methods and systems to implement the present disclosure, as well as non-transitory computer-readable recording medium recording having stored thereon computer programs for executing the methods.

According to the present disclosure, it is possible to make an estimation model learn so as to estimate biometric information about a head of a person to be measured based on NIRS and in consideration of an anatomical structure of a head portion of the person to be measured, and analyze an optical signal detected from the head portion of the person to be measured using the learned estimation model, thereby accurately and continuously monitoring the biometric information about the head of the person to be measured and increasing the cost-effectiveness of the monitoring.

BRIEF DESCRIPTION OF THE DRAWINGS

FIG. 1 illustratively shows a schematic configuration of an entire system for estimating biometric information about a head using an estimation model according to one embodiment of the present disclosure.
FIG. 2 illustratively shows a detailed internal configuration of a biometric information estimation system according to one embodiment of the present disclosure.
FIG. 3 exemplarily shows a process of obtaining an analysis target optical signal detected from a head portion of a person to be measured according to one embodiment of the present disclosure.
FIGS. 4 and 5 show visually represented data on an anatomical structure of a head portion according to one embodiment of the present disclosure.
FIG. 6 schematically shows an operation of the entire system for estimating biometric information about a head using an estimation model according to one embodiment of the present disclosure.

EXPLANATION OF REFERENCE NUMERALS

100: communication network
200: biometric information estimation system
210: analysis target optical signal acquisition unit
220: estimation model management unit
230: biometric information estimatibg unit
240: communication unit
250: control unit
300: device

DETAILED DESCRIPTION

In the following detailed description of the present disclosure, references are made to the accompanying drawings that show, by way of illustration, specific embodiments in which the present disclosure may be practiced. These embodiments are described in sufficient detail to enable those skilled in the art to practice the present disclosure. It is to be understood that the various embodiments of the present disclosure, although different from each other, are not necessarily mutually exclusive. For example, specific shapes, structures and characteristics described herein may be implemented as modified from one embodiment to another without departing from the scope of the present disclosure. Furthermore, it shall be understood that the positions or arrangements of individual elements within each of the disclosed embodiments may also be modified without departing from the scope of the present disclosure. Therefore, the following detailed description is not to be taken in a limiting sense, and the scope of the present invention, if properly described, is limited only by the appended claims. In the drawings, like reference numerals refer to the same or similar functions throughout the several views.

Hereinafter, preferred embodiments of the present disclosure will be described in detail with reference to the accompanying drawings to enable those skilled in the art to easily implement the present disclosure.

Configuration of Entire System

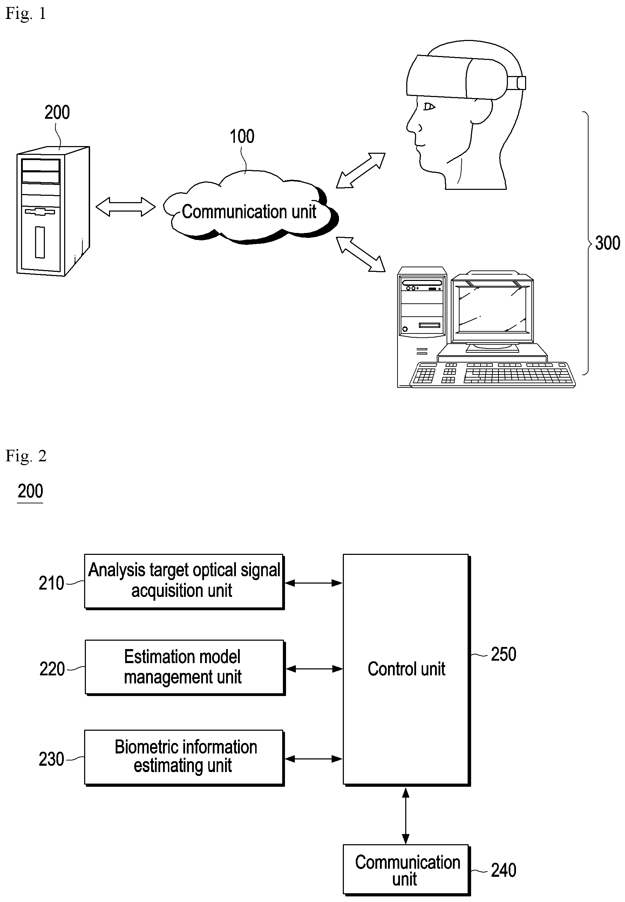

FIG. 1 illustratively shows a schematic configuration of an entire system for estimating biometric information about a head using an estimation model according to one embodiment of the present disclosure.

As shown in FIG. 1, the entire system according to one embodiment of the present disclosure may include a communication network 100, a biometric information estimation system 200, and a device 300.

The communication network 100 according to one embodiment of the present disclosure may be configured without taking a usual aspect such as wired or wireless communication into account, and may include a variety of communication networks such as a local area network (LAN), a metropolitan area network (MAN), a wide area network (WAN), and the like. Preferably, the communication network 100 described herein may be the Internet or the World Wide Web (WWW) which are well known. However, the communication network 100 is not necessarily limited thereto, and may at least partially include known wired/wireless data communication networks, known telephone networks, or known wired/wireless television communication networks.

For example, the communication network 100 may be a wireless data communication network, at least a portion of which may be implemented with a conventional communication scheme, such as wireless fidelity (WiFi) communication, WiFi-direct communication, Long Term Evolution (LTE) communication, 5G communication, Bluetooth communication (including Bluetooth Low Energy (BLE) communication), infrared communication, ultrasonic communication, or the like. As another example, the communication network 100 may be an optical communication network, and at least a portion of which may be implemented with a conventional communication scheme, such as light fidelity (LiFi) communication.

The biometric information estimation system 200 according to one embodiment of the present disclosure may perform a function of: acquiring an analysis target optical signal detected from a head portion of a person to be measured by at least one optical sensor disposed on the head portion of the person to be measured; and estimating the biometric information about the head of the person to be measured by analyzing the acquired analysis target optical signal using an estimation model learned based on data about an anatomical structure of at least one head portion, data about a biometrical state of the at least one head portion, and data about an optical signal associated with the data about the anatomical structure of at least one head portion and the data about the biometrical state of the at least one head portion.

A configuration and function of the biometric information estimation system 200 according to the present disclosure will be described in detail by the following detailed description.

The device 300 according to one embodiment of the present disclosure is a digital device that has a function of being connected to and communicate with the biometric information estimation system 200. Other digital devices may be employed as the device 300 according to the present disclosure as long as they include a memory means and a microprocessor for computing capabilities like a smartphone, a tablet, a smart watch, a smart band, a smart glass, a desktop computer, a notebook computer, a workstation, a PDA, a web pad, a mobile phone, and the like.

Specifically, the device 300 according to one embodiment of the present disclosure may include at least one optical sensor which performs a function of irradiating near-infrared rays to a head portion of a person to be measured and detecting the near-infrared rays reflected on or scattered from the head portion (more specifically, cerebral region) of the person to be measured. The device 300 may be configured in the form of a patch that can be attached to the head portion of the person to be measured. Here, one optical sensor may include at least one light irradiation part and at least one light detection part. Further, the device 300 according to one embodiment of the present disclosure may include a display means for providing a user with various information about the biometric information estimated by the biometric information estimating unit 230 according to one embodiment of the present disclosure.

In addition, according to one embodiment of the present disclosure, the device 300 may further include an application program for executing functions of the present disclosure. Such an application may be stored in the device 300 in the form of a program module. Features of the program modules may be generally similar to those of the analysis target optical signal acquisition unit 210, the estimation model management unit 220, the biometric information estimating unit 230, the communication unit 240, and the control unit 250 of the biometric information estimation system 200 which will be described below. Here, at least a portion of the application may be replaced with a hardware device or a firmware device that can perform a substantially same or equivalent function, as necessary.

Configuration of Biometric Information Estimation System

An internal configuration of the biometric information estimation system 200 which performs important functions to implement the present disclosure and functions of elements of the biometric information estimation system 200 will be described below.

FIG. 2 illustratively shows a detailed internal configuration of the biometric information estimation system 200 according to one embodiment of the present disclosure.

As shown in FIG. 2, the biometric information estimation system 200 according to one embodiment of the present disclosure may be configured to include the analysis target optical signal acquisition unit 210, the estimation model management unit 220, the biometric information estimating unit 230, the communication unit 240, and the control unit 250. According to one embodiment of the present disclosure, at least one among the analysis target optical signal acquisition unit 210, the estimation model management unit 220, the biometric information estimating unit 230, the communication unit 240, and the control unit 250 may be program modules configured to communicate with an external system. Such program modules may be included in the biometric information estimation system 200 in the form of operating systems, application program modules, and other program modules, while they may be physically stored in a variety of known storage devices. Further, the program modules may also be stored in a remote storage device that can communicate with the biometric information estimation system 200. Meanwhile, the program modules may include, but are not limited to, routines, subroutines, programs, objects, components, data structures, and the like for performing specific tasks or executing specific abstract data types as will be described below according to the present disclosure.

Although the biometric information estimation system 200 has been described as above, such a description is illustrative, and it will be apparent to those skilled in the art that at least a part of the components or functions of the biometric information estimation system 200 may be implemented inside or included in the device 300 as a wearable device which is to be worn on the head portion of the person to be measured, as necessary.

The analysis target optical signal acquisition unit 210 according to one embodiment of the present disclosure can perform a function of obtaining an analysis target optical signal detected from a head portion of a person to be measured by at least one optical sensor disposed on a head portion of the person to be measured.

Specifically, the device 300 according to one embodiment of the present disclosure may be worn on the head portion of the person to be measured, and include at least one optical sensor. Further, the analysis target optical signal acquisition unit 210 according to one embodiment of the present disclosure may acquire an analysis target optical signal detected from the head portion of the person to be measured by the at least one optical sensor. According to one embodiment of the present disclosure, the analysis target optical signal may refer to a signal of light intensity detected using near-infrared spectroscopy (NIRS).

For example, the analysis target optical signal acquisition unit 210 according to one embodiment of the present disclosure may acquire the analysis target optical signal of the person to be measured from the device 300 connected through a wireless communication network (e.g., a known local area network such as Wi-Fi, Wi-Fi Direct, LTE Direct, Bluetooth).

As another example, the analysis target optical signal acquisition unit 210 according to one embodiment of the present disclosure may acquire an analysis target optical signal for the person to be measured from at least one recording device (e.g., a server, a cloud, or the like) in which the analysis target optical signal is stored in advance.

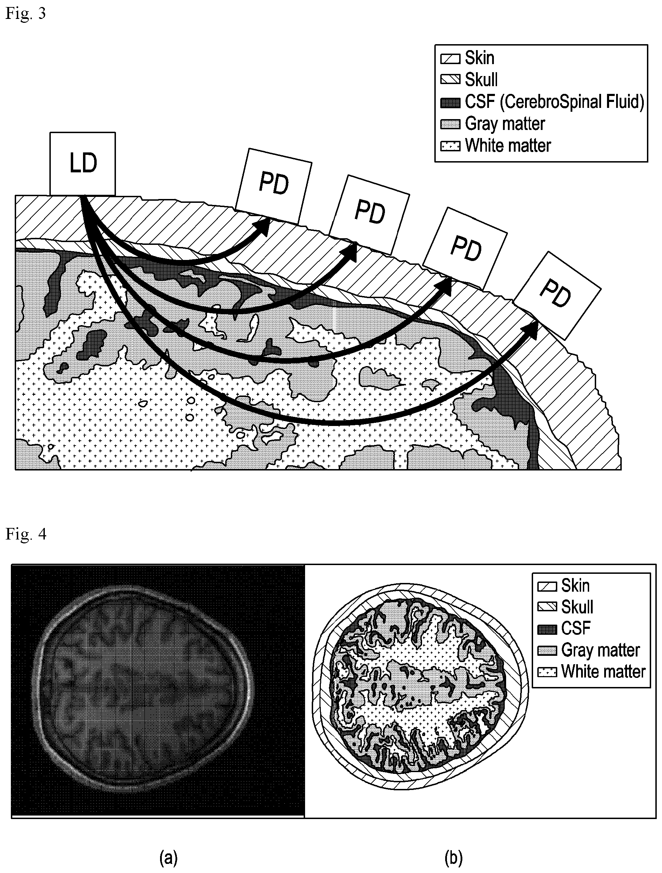

FIG. 3 exemplarily shows a process of acquiring the analysis target optical signal detected from the head portion of the person to be measured according to one embodiment of the present disclosure.

Referring to FIG. 3, it can be noted that a light driver (LD) included in the device 300 according to one embodiment of the present disclosure irradiates the head portion of the person to be measured with near-infrared rays, and a photo detector (PD) included in the device 300 according to one embodiment of the present disclosure detects the near-infrared rays reflected on or scattered from the head portion of the person to be measured. In addition, the analysis target optical signal acquisition unit 210 according to one embodiment of the present disclosure may acquire the detected near-infrared rays as an analysis target optical signal.

Further, the analysis target optical signal acquisition unit 210 according to one embodiment of the present disclosure may perform a function of managing the device 300 such that the at least one optical sensor included in the device 300 irradiates the head portion of the person to be measured with the near-infrared rays and detects the near-infrared rays reflected on or scattered from the head portion of the person to be measured. Furthermore, the analysis target optical signal acquisition unit 210 according to one embodiment of the present disclosure may manage other functions or components of the device 300 required to acquire the analysis target optical signal from the head portion of the person to be measured.

The estimation model management unit 220 according to one embodiment of the present disclosure may perform a function of making an estimation model learn based on data about an anatomical structure of at least one head portion, data about a biometrical state of the at least one head portion, and data about an optical signal associated with the data about the anatomical structure of the at least one head portion and the data about the biometrical state of the at least one head portion.

Here, the estimation model according to one embodiment of the present disclosure may be implemented using an artificial neural network, such as a convolutional neural network (CNN), a recurrent neural network (RNN), or the like, and optionally, may be implemented using a residual block. Furthermore, the estimation model according to one embodiment of the present disclosure may be implemented using other types of machine learning techniques instead of the artificial neural network. It should be understood that a technique capable of being used to implement and make the estimation model learn is not necessarily limited to the above examples, but may be variously modified as long as it can achieve the objects of the present disclosure.

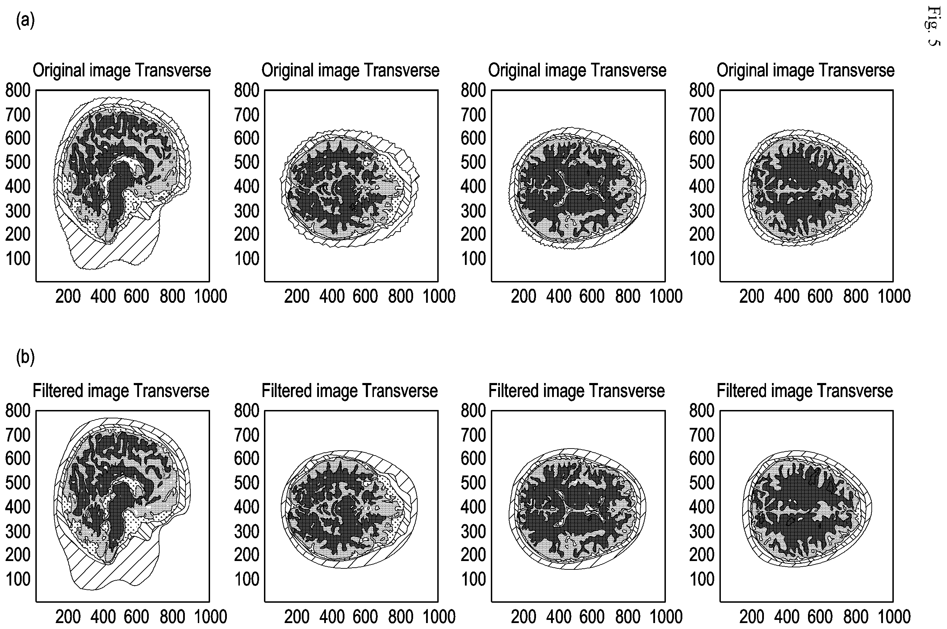

FIGS. 4 and 5 show visually represented data about the anatomical structure of the head portion according to an embodiment of the present disclosure.

Referring to FIG. 4 (a) the data about the anatomical structure of the head portion according to one embodiment of the present disclosure may refer to data including information about an anatomical layer of the head portion (e.g., information about a thickness, volume, or the like of each layer of the head portion), such as magnetic resonance imaging (MRI) data. Further, the data about the anatomical structure according to one embodiment of the present disclosure may refer to data pre-processed by the estimation model management unit 220 according to one embodiment of the present disclosure before input to a simulation model in order to improve the accuracy of the simulation model used to acquire data about the optical signal. Examples of the pre-process may include segmentation for distinguishing the anatomical layer of the head portion, smoothing for gently expressing the boundary of each layer of the segmented image, and the like.

For example, referring to FIG. 4 (b), there is illustrated a segmented MRI image in which five anatomical layers of the head portion (i.e., skin, skull, cerebrospinal fluid (CSF), gray matter, and white matter) are indicated with different gray-scaled colors in order to distinguish them from each other. Referring to FIG. 5, the result (FIG. 5 (b)) obtained by smoothing the segmented MRI image (FIG. 5 (a)) is illustrated.

The data about the anatomical structure of the head portion according to one embodiment of the present disclosure may be associated with the data about the biometrical state of the head portion. For example, the data about the biometrical state of the head portion according to one embodiment of the present disclosure may include information about the oxygen saturation of each layer of the head portion, information about hemoglobin concentration of each layer of the head portion (e.g., concentration of oxy hemoglobin, concentration of deoxy hemoglobin, concentration of hemoglobin, or the like), information about moisture content of each layer of the head portion, information about a degree of blood rushing to the head portion, information about the degree of flush, various disorders of the head portion (stroke, brain edema, Alzheimer's disease, and the like), and the like. Further, the data about the biometrical state of the head portion according to one embodiment of the present disclosure may be associated with data about the anatomical structure of the head portion in a format such as meta data. However, the type of information included in the data about the biometrical state of the head portion and the format in which the data about the anatomical structure of the head portion is associated with the data about the anatomical structure of the head portion are not limited to the above examples, but may be variously modified as long as it can achieve the objects of the present disclosure.

The data about the anatomical structure of the head portion according to one embodiment of the present disclosure may be generated based on an optical property coefficient assigned to each anatomical layer of the head portion.

Specifically, the estimation model management unit 220 according to one embodiment of the present disclosure may assign, to each anatomical layer of the head portion, an optical property coefficient associated with a biometrical state of each anatomical layer. Furthermore, the estimation model management unit 220 according to one embodiment of the present disclosure may divide one layer into two or more regions (e.g., divide the left brain region and the right brain region), even if the regions are included in one layer, and assign an optical property coefficient to each of the divided regions. Then, the estimation model management unit 220 according to one embodiment of the present disclosure may generate data about the anatomical structure of the head portion based on the assigned optical property coefficient.

Examples of the optical property coefficient assigned by the estimation model management unit 220 according to one embodiment of the present disclosure may include an absorption coefficient $\mu_a$, a reduced scattering coefficient $\mu_s'$, and the like. A range (boundary condition) of the optical property coefficient assigned as above may be limited to a range that may appear in the person's body. In addition, the optical property coefficient assigned as above may be determined according to a biometrical state of each anatomical layer of the head portion. On the contrary, the biometrical state of each anatomical layer of the head portion may be determined according to an optical property coefficient arbitrarily assigned by the estimation model management unit 220 according to one embodiment of the present disclosure.

According to one embodiment of the present disclosure, by assigning various values of optical property coefficients (e.g., a combination of various values of adsorption coefficients and various values of reduced scattering coefficients) to each anatomical layer of the head portion, data about an anatomical structure of the head portion having the various optical property coefficients may be generated from data (e.g., one MRI image data) about an anatomical structure of one head portion. As a result, a lot of data for making the estimation model learn can be obtained.

The data about the optical signal according to one embodiment of the present disclosure may be associated with the data about the anatomical structure of the head portion and the data about the biometrical state of the head portion according to one embodiment of the present disclosure.

Specifically, when it is assumed that at least one optical sensor is disposed on the head portion, the estimation model management unit 220 according to one embodiment of the present disclosure may predict light intensity that can be detected from the head portion by using a simulation model in which the data about the anatomical structure of the head portion and the data about the biometrical state of the head portion are used as input data, and obtain data about the optical signal. That is, the data about the obtained optical signal, which is output data of the simulation model, may be used as labeled data in making the estimation model learn according to one embodiment of the present disclosure. Here, it should be understood that the above-described optical sensor is virtually provided, and the number or form of virtually-provided optical sensors may be variously changed.

As an example, the simulation model according to one embodiment of the present disclosure may be a simulation model based on the Monte-Carlo method. This simulation model may predict the intensity of light that can be detected from the head portion by individually tracking photons that can be irradiated by the virtually-provided optical sensor.

The biometric information estimating unit 230 according to one embodiment of the present disclosure may use the estimation model learned using the estimation model management unit 220 according to one embodiment of the present disclosure to analyze an analysis target optical signal acquired by the analysis target optical signal acquisition unit 210 according to one embodiment of the present disclosure and estimate biometric information about the head of the person to be measured.

Specifically, the biometric information about the head estimated by the biometric information estimating unit 230 according to one embodiment of the present disclosure may include at least one of information about the effective attenuation coefficient $\mu_{eff}$ of the cerebral cortex, information about the oxygen saturation of the cerebral cortex, information about the volume of the cerebrospinal fluid, information about moisture content in the cerebral cortex, and information about the anatomical structure. Each information described above may include a time-dependent information change. Further, each of the estimated biometric information about the head may refer to an accurate numerical value of each estimated biometric information about the head. In this case, a known regression technique may be used. Furthermore, each of the estimated biometric information about the head may refer to whether or not the value of each estimated biometric information about the head exceeds a predetermined threshold value. In this case, a known classification technique may be used.

More specifically, according to one embodiment of the present disclosure, information about the effective attenuation coefficient of the cerebral cortex may include values of the effective attenuation coefficient $\mu_{eff}$, the absorption coefficient $\mu_a$, and the reduced scattering coefficient $\mu_s'$, and the like. The effective attenuation coefficient may be defined as follow:

$$\sqrt{3\mu_a\mu_s'} \text{ or } \sqrt{3\mu_a(\mu_a+\mu_s')}$$

Further, when light of a single wavelength is irradiated to the head portion of the person to be measured from the optical sensor according to one embodiment of the present disclosure and an analysis target optical signal is acquired through the detection of the light, the information about the effective attenuation coefficient of the cerebral cortex may be estimated by analyzing the analysis target optical signal obtained from the biometric information estimating unit 230 according to one embodiment of the present disclosure by using the estimation model.

According to one embodiment of the present disclosure, the oxygen saturation of the cerebral cortex may be defined as (concentration of oxy hemoglobin of cerebral cortex) /(concentration of oxy hemoglobin of cerebral cortex + concentration of deoxy hemoglobin of cerebral cortex). Further, when light of a plurality of wavelengths is irradiated to the head portion of the person to be measured from the optical sensor according to one embodiment of the present disclosure and an analysis target optical signal is acquired through the detection of the light, the information about the oxygen saturation of the cerebral cortex may be estimated by analyzing the analysis target optical signal obtained from the biometric information estimating unit 230 according to one embodiment of the present disclosure by using the estimation model. Furthermore, the information about the oxygen saturation of the cerebral cortex may be calculated based on information about the effective attenuation coefficient of the cerebral cortex estimated from each of the plurality of wavelengths. In some embodiments, it is also possible to directly estimate the information about the oxygen saturation of the cerebral cortex using the estimation model without undergoing the calculation process.

According to one embodiment of the present disclosure, the information about the volume of the cerebrospinal fluid may refer to the volume of the cerebrospinal fluid under an area where the head portion of the person to be measured wears the device 300 according to one embodiment of the present disclosure, but the present disclosure is not limited thereto. Further, when light of a plurality of wavelengths is irradiated to the head portion of the person to be measured from the optical sensor according to one embodiment of the present disclosure and an analysis target optical signal is acquired through the detection of the light, the information about the volume of the cerebrospinal fluid may be estimated by analyzing the analysis target optical signal obtained from the biometric information estimating unit 230 according to one embodiment of the present disclosure by using the estimation model .

According to one embodiment of the present disclosure, the information about the moisture content in the cerebral cortex may include moisture content included in at least a portion of the cerebral cortex, a ratio of the volume of at least a portion of the cerebral cortex to the volume of moisture contained in the corresponding region, a value obtained by dividing the ratio by the hemoglobin concentration, and the like. Further, when light of a plurality of wavelengths is irradiated to the head portion of the person to be measured from the optical sensor according to one embodiment of the present disclosure and an analysis target optical signal is acquired through the detection of the light, the information about the moisture content in the cerebral cortex may be estimated by analyzing the analysis target optical signal obtained from the biometric information estimating unit 230 according to one embodiment of the present disclosure by using the estimation model.

According to one embodiment of the present disclosure, the information about the anatomical structure may include a form and a thickness of each anatomical layer of the head portion (i.e., a thickness of the scalp, a thickness of the skull, a thickness of the gray matter, or the like).

The analysis target optical signal obtained by the analysis target optical signal acquisition unit 210 according to one embodiment of the present disclosure may include a first analysis target optical signal acquired from a first optical sensor disposed on a first region of the head portion of the person to be measured, and a second analysis target optical signal acquired from a second optical sensor disposed on a second region of the head portion of the person to be measured. Further, according to one embodiment of the present disclosure, the biometric information estimating unit 230 may estimate the biometric information of the first region relating to the head of the person to be measured by analyzing the acquired first analysis target optical signal using the estimation model, and may estimate the biometric information of the second region relating to the head of the person to be measured by analyzing the acquired second analysis target optical signal using the estimation model.

The biometric information of the first region and the biometric information of the second region relating to the head of the person to be measured, which are estimated by the biometric information estimating unit 230 according to one embodiment of the present disclosure, may include at least one of the information about the effective attenuation coefficient $\mu_{eff}$ of the cerebral cortex and the information about the oxygen saturation of the cerebral cortex. Further, the biometric information estimating unit 230 according to one embodiment of the present disclosure may calculate third biometric information about the head of the person to be measured by comparing the biometric information of the first region with the biometric information of the second region. Here, according to one embodiment of the present disclosure, the third biometric information calculated as described above may refer to a difference between the biometric information of the first region and the biometric information of the second region.

Each estimated biometric information about the head (e.g., each of the effective attenuation coefficient of the first region, the effective attenuation coefficient of the second region, and a difference between the effective attenuation coefficient of the first region and the effective attenuation coefficient of the second region (i.e., the third biometric information)) may refer to a numerical value of each estimated biometric information about the head. In this case, a known regression technique may be used. Further, each estimated biometric information about the head may refer to whether or not the value of each estimated biometric information about the head exceeds a predetermined threshold value. In this case, a known classification technique may be used.

For example, according to one embodiment of the present disclosure, it is assumed that a first optical sensor is disposed on a left region (i.e., the first region) of the head portion of the person to be measured, and a second optical sensor is disposed on a right region (i.e., the second region) of the head portion of the person to be measured. In this case, the analysis target optical signal acquisition unit 210 according to one embodiment of the present disclosure may acquire an analysis target optical signal of the person to be measured in each region. Then, the biometric information estimating unit 230 according to one embodiment of the present disclosure may estimate the biometric information of the first region and the biometric information of the second region relating to the head of the person to be measured in each region by analyzing the above acquired analysis target optical signal of each region using the estimation model. Substantially, the biometric information estimating unit 230 according to one embodiment of the present disclosure may calculate the third biometric information about the head of the person to be measured by calculating the difference between the biometric information of the first region and the biometric information of the second region. As described above, the method of acquiring the analysis target optical signal by dividing the region of the head portion of the person to be measured into two or more regions may be useful when a disorder occurs only in some of the two or more regions of the head portion of the person to be measured (e.g., a stroke occurred in the right brain) or when the occurrence of such a disorder is expected.

The above description regarding estimation of the biometric information of the first region and the biometric information of the second region is not limited thereto, but may be applied to estimation of the biometric information of two or more regions. That is, according to one embodiment of the present disclosure, a first analysis target optical signal, a second analysis target optical signal, ..., and an n-th analysis target optical signal may be acquired from respective optical sensors disposed on a first region, a second region,...,and an n-th region of the head portion of the person to be measured, and biometric information of the first region, biometric information of the second region, ..., and biometric information of the n-th region relating to the head of the person to be measured may be estimated by analyzing each of the analysis target optical signals. Further, biometric information about the head of the person to be measured may be calculated by comparing the biometric information of the first region, the biometric information of the second region, ..., and the biometric information of the n-th region with each other.

When the biometric information about the head of the person to be measured is estimated by the biometric information estimating unit 230 according to one embodiment of the present disclosure, the biometric information estimating unit 230 according to one embodiment of the present disclosure, which is not covered by the present invention as claimed, may determine a biometric state relating to the cerebrum of the person to be measured based on the estimated biometric information. The biometric state relating to the cerebrum of the person to be measured may include information about the cerebral apoplexy, cerebral edema, Alzheimer's disease, or the like, but the present disclosure is not limited thereto.

As an example, based on at least one of the information about the effective attenuation coefficient of the cerebral cortex and the information about the oxygen saturation of the cerebral cortex of the person to be measured estimated by the biometric information estimating unit 230 according to one non-claimed embodiment of the present disclosure, it may be determined whether the person to be measured is in a stroke state or at risk of having a stroke.

As another example, based on the information about the volume of the cerebrospinal fluid of the person to be measured estimated by the biometric information estimating unit 230 according to one non-claimed embodiment of the present disclosure, it may be determined whether the person to be measured has Alzheimer's disease or is at risk of having such a disease.

As yet another example, based on the information about the moisture content in the cerebral cortex of the person to be measured estimated by the biometric information estimating unit 230 according to one non-claimed embodiment of the present disclosure, it may be determined whether the person to be measured is in a cerebral edema state or at risk of having such a disease.

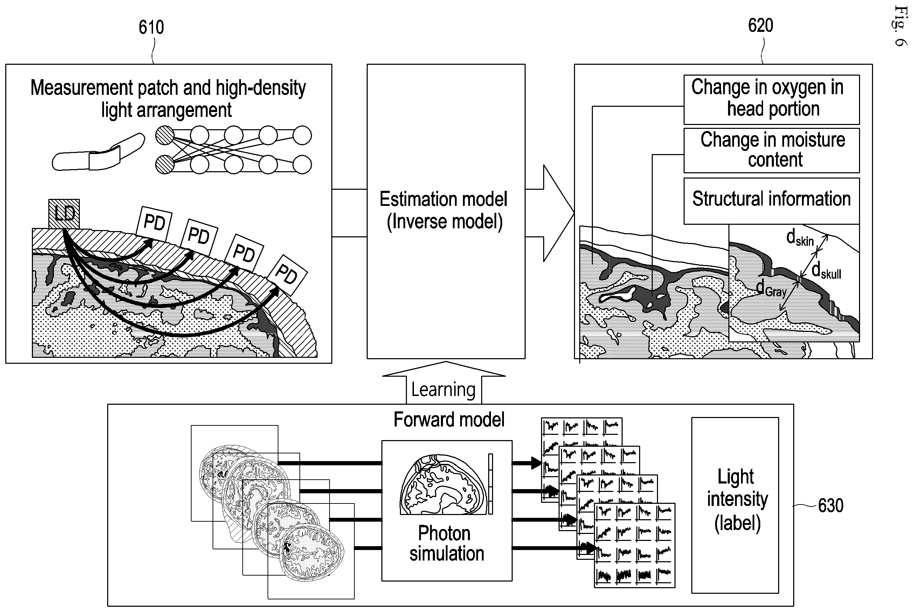

FIG. 6 schematically shows an operation of the entire system for estimating biometric information about the head using a machine learning, according to one embodiment of the present disclosure.

Referring to FIG. 6, the estimation model management unit 220 according to one embodiment of the present disclosure may use a simulation model in which data about an anatomical structure of at least one head portion and data about a biometrical state of the at least one head portion are used as input data, to acquire data about optical signals associated with the data about the anatomical structure of the at least one head portion and the data about the biometric state of the at least one head portion (in 630).

Further, referring to FIG. 6, the estimation model management unit 220 according to one embodiment of the present disclosure may make the estimation model learn according to one embodiment of the present disclosure based on the data about the anatomical structure of the at least one head portion, the data about the biometric state of the at least one head portion, and the data about the optical signals associated with the data about the anatomical structure of the at least one head portion and the data about the biometric state of the at least one head portion.

Further, referring to FIG. 6, the analysis target optical signal acquisition unit 210 according to one embodiment of the present disclosure may acquire an analysis target optical signal detected from the head portion of the person to be measured by at least one optical sensor disposed on the head portion of the person to be measured (in 610). Subsequently, the biometric information estimating unit 230 according to one embodiment of the present disclosure may estimate the biometric information about the head of the person to be measured by analyzing the acquired analysis target optical signal using the estimation model (in 620).

The communication unit 240 according to one embodiment of the present disclosure may perform a function of enabling transmission/reception of data to/from the analysis target optical signal acquisition unit 210, the estimation model management unit 220, and the biometric information estimating unit 230.

The control unit 250 according to one embodiment of the present disclosure may function to control the flow of data among the analysis target optical signal acquisition unit 210, the estimation model management unit 220, the biometric information estimating unit 230, and the communication unit 240. That is, the control unit 250 according to the present disclosure may control the flow of data from/to the outside of the biometric information estimation system 200, or the flow of data between respective components of the biometric information estimation system 200, such that the analysis target optical signal acquisition unit 210, the estimation model management unit 220, the biometric information estimating unit 230, and the communication unit 240 may carry out their particular functions, respectively.

The embodiments according to the present disclosure as described above may be implemented in the form of program commands that can be executed by various computer components, and may be recorded on a computer-readable recording medium. The computer-readable recording medium may include program commands, data files, and data structures, independently or in combination. The program commands recorded on the computer-readable recording medium may be specially designed and configured for the present disclosure, or may also be well known and available to and by those skilled in the computer software field. Examples of the non-transitory computer-readable recording medium include the following: magnetic media such as hard disks, floppy disks and magnetic tapes; optical media such as compact disk-read only memory (CD-ROM) and digital versatile disks (DVDs); magneto-optical media such as floptical disks; and hardware devices such as read-only memory (ROM), random access memory (RAM) and flash memory, which are specially configured to store and execute program instructions. Examples of the program commands include not only machine language codes created by a compiler, but also high-level language codes that can be executed by a computer using an interpreter. The above hardware devices may be changed to one or more software modules to perform the processes of the present disclosure, and vice versa.

Although the present disclosure has been described above in terms of specific items such as detailed constituent elements as well as the limited embodiments and the drawings, they are only provided to help more general understanding of the invention, and the present disclosure is not limited to the above embodiments. It will be appreciated by those skilled in the art to which the present disclosure pertains that various modifications and changes may be made from the above description.

## Claims

1. A method of estimating biometric information about a head using a machine learning, the method comprising:

   acquiring, by an analysis target optical signal acquisition unit (210), an analysis target optical signal detected from a head portion of a person to be measured by at least one optical sensor disposed on the head portion of the person to be measured; and
   estimating, by a biometric information estimating unit (230), the biometric information about the head of the person to be measured by analyzing the acquired analysis target optical signal using an estimation model learned, by an estimation model management unit (220), based on data about an anatomical structure of at least one head portion, data about a biometrical state of the at least one head portion, and data about an optical signal associated with the data about the anatomical structure of at least one head portion and the data about the biometrical state of the at least one head portion,
   **characterized in that** the data about the optical signal is acquired using a simulation model in which the data about the anatomical structure of the at least one head portion and the data about the biometrical state of the at least one head portion are used as input data, and the data about the optical signal is used as labeled data in making the estimation model learn.

2. The method of Claim 1, wherein the data about the anatomical structure of the at least one head portion is generated based on a light property coefficient assigned to each anatomical layer of the at least one head portion.

3. The method of Claim 1, wherein the estimated biometric information includes at least one of information about an effective attenuation coefficient of a cerebral cortex, information about an oxygen saturation of the cerebral cortex, information about a volume of a cerebrospinal fluid, information about moisture content of the cerebral cortex, and information about the anatomical structure.

4. The method of Claim 1, wherein, in the acquiring step, the analysis target optical signal comprises a first analysis target optical signal acquired from a first optical sensor disposed on a first region of the head portion of the person to be measured and a second analysis target optical signal acquired from a second optical sensor disposed on a second region of the head portion of the person to be measured, and
   in the estimating step, biometric information of the first region of the head of the person to be measured is estimated by analyzing the acquired first analysis target optical signal using the estimation model, and biometric information of the second region of the head of the person to be measured is estimated by analyzing the acquired second analysis target optical signal using the estimation model.

5. The method of Claim 4, wherein the biometric information of the first region and the biometric information of the second region comprise at least one of information about an effective attenuation coefficient of a cerebral cortex and information about an oxygen saturation of the cerebral cortex,
   in the estimating step, a third biometric information about the head of the person to be measured is calculated by comparing the biometric information of the first region and the biometric information of the second region with each other.

6. The method of Claim 1, wherein the analysis target optical signal is acquired using near-infrared spectroscopy (NIRS).

7. The method of Claim 1, wherein the optical sensor comprises at least one light irradiation part and at least one light detection part.

8. The method of Claim 1, wherein the data about the anatomical structure of the at least one head portion is pre-processed before input to the simulation model.

9. A non-transitory computer-readable recording medium having stored thereon a computer program for executing the method of Claim 1.

10. A system (200) for estimating biometric information about a head by using a machine learning, comprising:

an analysis target optical signal acquisition unit (210) configured to acquire an analysis target optical signal detected from a head portion of a person to be measured by at least one optical sensor disposed on the head portion of the person to be measured;
an estimation model management unit (220) configured to make an estimation model learn based on data about an anatomical structure of at least one head portion, data about a biometrical state of the at least one head portion, and data about an optical signal associated with the data about the anatomical structure of at least one head portion and the data about the biometrical state of the at least one head portion; and
a biometric information estimating unit (230) configured to estimate the biometric information about the head of the person to be measured by analyzing the acquired analysis target optical signal using the learned estimation model learned,
**characterized in that** the estimation model management unit (220) is configured to acquire the data about the optical signal by using a simulation model in which the data about the anatomical structure of the head portion and the data about the biometrical state of the head portion are used as input data, and the data about the optical signal is used as labeled data in making the estimation model learn.

**Patentansprüche**

1. Verfahren zum Schätzen biometrischer Informationen über einen Kopf unter Verwendung eines maschinellen Lernens, wobei das Verfahren Folgendes umfasst:

Erfassen, durch eine optische Analysezielsignal-Erfassungseinheit (210), eines optischen Analysezielsignals, das von einem Kopfabschnitt einer zu messenden Person durch mindestens einen optischen Sensor erkannt wird, der an dem Kopfabschnitt der zu messenden Person angebracht ist; und
Schätzen, durch eine Schätzeinheit für biometrische Informationen (230), der biometrischen Informationen über den Kopf der zu messenden Person durch Analysieren des erfassten optischen Analysezielsignals unter Verwendung eines Schätzmodells, das durch eine Schätzmodell-Verwaltungseinheit (220) anhand von Daten über eine anatomische Struktur mindestens eines Kopfabschnitts, von Daten über einen biometrischen Zustand des mindestens einen Kopfabschnitts und von Daten über ein optisches Signal, das den Daten über die anatomische Struktur des mindestens einen Kopfabschnitts und den Daten über den biometrischen Zustand des mindestens einen Kopfabschnitts zugeordnet ist, ermittelt wurde,
**dadurch gekennzeichnet, dass** die Daten über das optische Signal unter Verwendung eines Simulationsmodells erfasst werden, in dem die Daten über die anatomische Struktur des mindestens einen Kopfabschnitts und die Daten über den biometrischen Zustand des mindestens einen Kopfabschnitts als Eingabedaten verwendet werden, und die Daten über das optische Signal als gekennzeichnete Daten verwendet werden, um das Schätzmodell ermitteln zu lassen.

2. Verfahren nach Anspruch 1, wobei die Daten über die anatomische Struktur des mindestens einen Kopfabschnitts anhand eines Lichteigenschaftskoeffizienten erzeugt werden, der jeder anatomischen Schicht des mindestens einen Kopfabschnitts zugeordnet ist.

3. Verfahren nach Anspruch 1, wobei die geschätzten biometrischen Informationen mindestens eine der folgenden Informationen umfassen: Informationen über einen effektiven Dämpfungskoeffizienten einer Großhirnrinde, Informationen über eine Sauerstoffsättigung der Großhirnrinde, Informationen über ein Volumen einer zerebrospinalen Flüssigkeit, Informationen über den Feuchtigkeitsgehalt der Großhirnrinde und Informationen über die anatomische Struktur.

4. Verfahren nach Anspruch 1, wobei, in dem Erfassungsschritt, das optische Analysezielsignal ein erstes optisches Analysezielsignal umfasst, das von einem ersten optischen Sensor erfasst wird, der an einem ersten Bereich des Kopfabschnitts der zu messenden Person angebracht ist, und ein zweites optisches Analysezielsignal, das von einem zweiten optischen Sensor erfasst wird, der an einem zweiten Bereich des Kopfabschnitts der zu messenden Person angebracht ist, und
in dem Schätzungsschritt biometrische Informationen des ersten Bereichs des Kopfes der zu messenden Person durch Analysieren des erfassten ersten optischen Analysezielsignals unter Verwendung des Schätzmodells geschätzt werden, und biometrische Informationen des zweiten Bereichs des Kopfes der zu messenden Person durch Analysieren des erfassten zweiten optischen Analysezielsignals unter Verwendung des Schätzmodells geschätzt werden.

5. Verfahren nach Anspruch 4, wobei die biometrischen Informationen des ersten Bereichs und die biometrischen Informationen des zweiten Bereichs mindestens eine der folgenden Informationen umfassen: Informationen über einen effektiven Dämpfungskoeffizienten einer Großhirnrinde und Informationen über eine Sauerstoffsättigung der Großhirnrinde,
in dem Schätzungsschritt eine dritte biometrische Information über den Kopf der zu messenden Person durch Vergleichen der biometrischen Information des ersten Bereichs und der biometrischen Information des zweiten Bereichs miteinander berechnet wird.

6. Verfahren nach Anspruch 1, wobei das optische Analysezielsignal unter Verwendung von Nahinfrarotspektroskopie (NIRS) erfasst wird.

7. Verfahren nach Anspruch 1, wobei der optische Sensor mindestens einen Lichtbestrahlungsteil und mindestens ein Lichterkennungsteil umfasst.

8. Verfahren nach Anspruch 1, wobei die Daten über die anatomische Struktur des mindestens einen Kopfabschnitts vor der Eingabe in das Simulationsmodell vorverarbeitet werden.

9. Nichtflüchtiges, computerlesbares Aufzeichnungsmedium, auf dem ein Computerprogramm zum Ausführen des Verfahrens nach Anspruch 1 gespeichert ist.

10. System (200) zum Schätzen biometrischer Informationen über einen Kopf unter Verwendung eines maschinellen Lernens, umfassend:

eine optische Analysezielsignal-Erfassungseinheit (210), die so konfiguriert ist, dass sie ein optisches Analysezielsignal erfasst, das von einem Kopfabschnitt einer zu messenden Person durch mindestens einen optischen Sensor erkannt wird, der an dem Kopfabschnitt der zu messenden Person angebracht ist;
eine Schätzmodell-Verwaltungseinheit (220), die so konfiguriert ist, dass sie ein Schätzmodell anhand von Daten über eine anatomische Struktur mindestens eines Kopfabschnitts, von Daten über einen biometrischen Zustand des mindestens einen Kopfabschnitts und von Daten über ein optisches Signal, das den Daten über die anatomische Struktur des mindestens einen Kopfabschnitts und den Daten über den biometrischen Zustand des mindestens einen Kopfabschnitts zugeordnet ist, ermittelt,
eine Schätzeinheit für biometrische Informationen (230), die so konfiguriert ist, dass sie die biometrischen Informationen über den Kopf der zu messenden Person durch Analysieren des erfassten optischen Analysezielsignals unter Verwendung des ermittelten Schätzmodells schätzt,
**dadurch gekennzeichnet, dass** die Schätzmodell-Verwaltungseinheit (220) so konfiguriert ist, dass sie die Daten über das optische Signal unter Verwendung eines Simulationsmodells erfasst, in dem die Daten über die anatomische Struktur des Kopfabschnitts und die Daten über den biometrischen Zustand des Kopfabschnitts als Eingabedaten verwendet werden, und die Daten über das optische Signal als gekennzeichnete Daten verwendet werden, um das Schätzmodell ermitteln zu lassen.

**Revendications**

1. Procédé d'estimation d'informations biométriques concernant une tête au moyen d'un apprentissage machine, le procédé comprenant :

l'acquisition, par une unité d'acquisition de signal optique cible d'analyse (210), d'un signal optique cible d'analyse détecté à partir d'une partie de tête d'une personne à mesurer par au moins un capteur optique disposé sur la partie de tête de la personne à mesurer ; et
l'estimation, par une unité d'estimation d'informations biométriques (230), des informations biométriques concernant la tête de la personne à mesurer par analyse du signal optique cible d'analyse acquis au moyen d'un modèle d'estimation appris, par une unité de gestion de modèle d'estimation (220), sur la base de données concernant une structure anatomique d'au moins une partie de tête, de données concernant un état biométrique de l'au moins une partie de tête, et de données concernant un signal optique associé aux données concernant la structure anatomique de l'au moins une partie de tête et aux données concernant l'état biométrique de l'au moins une partie de tête,
**caractérisé en ce que** les données concernant le signal optique sont acquises au moyen d'un modèle de simulation dans lequel les données concernant la structure anatomique de l'au moins une partie de tête et les données concernant l'état biométrique de l'au moins une partie de tête sont utilisées comme données d'entrée, et les données concernant le signal optique sont utilisées comme données étiquetées dans l'apprentissage du modèle d'estimation.

2. Procédé selon la revendication 1, dans lequel les données concernant la structure anatomique de l'au moins une partie de tête sont générées sur la base d'un coefficient de propriété de lumière attribué à chaque couche anatomique de l'au moins une partie de tête.

3. Procédé selon la revendication 1, dans lequel les informations biométriques estimées incluent des informations concernant un coefficient d'atténuation efficace d'un cortex cérébral et/ou des informations concernant une saturation en oxygène du cortex cérébral et/ou des informations concernant un volume d'un liquide cérébro-spinal et/ou des informations concernant une teneur en humidité du cortex cérébral et/ou des informations concernant la structure anatomique.

4. Procédé selon la revendication 1, dans lequel, dans l'étape d'acquisition, le signal optique cible d'analyse comprend un premier signal optique cible d'analyse acquis auprès d'un premier capteur optique disposé sur une première région de la partie de tête de la personne à mesurer et un second signal optique cible d'analyse acquis auprès d'un second capteur optique disposé sur une seconde région de la partie de tête de la personne à mesurer, et dans l'étape d'estimation, des informations biométriques de la première région de la tête de la personne à mesurer sont estimées par l'analyse du premier signal optique cible d'analyse acquis au moyen du modèle d'estimation, et des informations biométriques de la seconde région de la tête de la personne à mesurer sont estimées par l'analyse du second signal optique cible d'analyse acquis au moyen du modèle d'estimation.

5. Procédé selon la revendication 4, dans lequel les informations biométriques de la première région et les informations biométriques de la seconde région comprennent des informations concernant un coefficient d'atténuation efficace d'un cortex cérébral et/ou des informations concernant une saturation en oxygène du cortex cérébral, dans l'étape d'estimation, des troisièmes informations biométriques concernant la tête de la personne à mesurer sont calculées en comparant les informations biométriques de la première région et les informations biométriques de la seconde région entre elles.

6. Procédé selon la revendication 1, dans lequel le signal optique cible d'analyse est acquis au moyen d'une spectroscopie proche infrarouge (NIRS).

7. Procédé selon la revendication 1, dans lequel le capteur optique comprend au moins une partie d'irradiation de lumière et au moins une partie de détection de lumière.

8. Procédé selon la revendication 1, dans lequel les données concernant la structure anatomique de l'au moins une partie de tête sont prétraitées avant l'entrée dans le modèle de simulation.

9. Support d'enregistrement non transitoire lisible par ordinateur sur lequel est stocké un programme informatique pour l'exécution du procédé selon la revendication 1.

10. Système (200) pour l'estimation d'informations biométriques concernant une tête au moyen d'un apprentissage machine, comprenant :

une unité d'acquisition de signal optique cible d'analyse (210) configurée pour acquérir un signal optique cible d'analyse détecté à partir d'une partie de tête d'une personne à mesurer par au moins un capteur optique disposé sur la partie de tête de la personne à mesurer ;
une unité de gestion de modèle d'estimation (220) configurée pour l'apprentissage d'un modèle d'estimation sur la base de données concernant une structure anatomique d'au moins une partie de tête, de données concernant un état biométrique de l'au moins une partie de tête, et de données concernant un signal optique associé aux données concernant la structure anatomique d'au moins une partie de tête et aux données concernant l'état biométrique de l'au moins une partie de tête ; et
une unité d'estimation d'informations biométriques (230) configurée pour estimer les informations biométriques concernant la tête de la personne à mesurer par l'analyse du signal optique cible d'analyse acquis au moyen du modèle d'estimation appris,
**caractérisé en ce que** l'unité de gestion de modèle d'estimation (220) est configurée pour acquérir les données concernant le signal optique au moyen d'un modèle de simulation dans lequel les données concernant la structure anatomique de la partie de tête et les données concernant l'état biométrique de la partie de tête sont utilisées comme données d'entrée, et les données concernant le signal optique sont utilisées comme données étiquetées dans l'apprentissage du modèle d'estimation.

Fig. 1

Fig. 2

200

Fig. 3

Fig. 4

(a)

(b)

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020160121348
- US 10071245 B1
- US 2017231501 A1

**Non-patent literature cited in the description**

- **FOURNIER MARC**. *Realistic Head Model Design and 3D Brain Imaging of NIRS Signals Using Audio Stimuli on Preterm Neonates for Intra Ventricular Hemorrhage Diagnosis*